# EUROPEAN PATENT SPECIFICATION

(11) **EP 4 545 055 B1**
(45) Date of publication and mention of the grant of the patent: **18.02.2026**
(21) Application number: 24205462.5
(22) Date of filing: 09.10.2024
(51) Int. Cl.: A61F 13/56, A61F 13/15

(54) **A CLOSING ELEMENT FOR PRODUCING ABSORBENT SANITARY ARTICLES**
VERSCHLUSSELEMENT ZUR HERSTELLUNG SAUGFÄHIGER HYGIENEARTIKEL
ELEMENT DE FERMETURE POUR LA PRODUCTION D'ARTICLES HYGIENIQUES ABSORBANTS

(30) Priority: 23.10.2023 IT 202300022089
(43) Date of publication of application: 30.04.2025
(73) Proprietor: Fameccanica.Data S.p.A., 66020 San Giovanni Teatino (CH) (IT)
(72) Inventor: SABLONE, Gabriele, 66020 San Giovanni Teatino (Chieti) (IT)
(74) Representative: Buzzi, Notaro & Antonielli d'Oulx S.p.A.

(56) References cited:
- EP-B1- 1 523 968

## Description

### Field of the invention

The invention relates to a closing element intended to be used for producing absorbent sanitary articles.

The invention was developed with particular attention to producing absorbent sanitary articles that can be worn like pants, such as so-called training pants, according to the production technique known as "Cross Direction".

### Description of the prior art

A well-established technique for producing wearable absorbent sanitary articles in the form of reclosable panty-type underwear consists of forming a continuous composite web movable in a machine direction, and formed of a continuous chain of article blanks arranged with their respective longitudinal axes transverse to the machine direction. This production technique called Cross Direction is different from the production technique called Machine Direction which involves producing the absorbent sanitary articles while they advance with their longitudinal axes parallel to the machine direction.

Examples of methods for producing absorbent sanitary articles according to the Cross Direction technique are described in documents EP-A-1013251, IT1379452, IT1410464 and IT1410465 by the same Applicant.

The continuous composite tape forming the chain of product blanks normally comprises two elastic bands parallel to the machine direction and spaced apart in a direction perpendicular to the machine direction, and a plurality of absorbent cores extending between the two elastic bands perpendicular to the machine direction. The continuous composite tape advancing in the machine direction is folded along a longitudinal axis parallel to the machine direction, so that the two elastic bands overlap each other.

For producing refastenable absorbent sanitary articles, refastenable closing elements are applied to the continuous composite tape, which are coupled with complementary closing elements after longitudinal folding of the tape.

EP-A-1523968 by the same applicant describes a closing element for producing absorbent sanitary articles wearable in the form of panties, comprising a non-woven sheet folded according to a general omega-shaped configuration and including a base subdivided into two sections located on opposite sides of a central plane, two intermediate flaps located on opposite sides of the central plane, connected to respective outer ends of the base sections and permanently fixed to the base, two distal flaps located on opposite sides of the central plane, connected to respective inner ends of the respective intermediate flaps by respective folds and fixed in a separable manner to respective intermediate flaps. Respective surface fasteners are fixed on surfaces of respective distal flaps opposite to the base. In one embodiment the lengths of the distal flaps are greater than the lengths of the intermediate flaps, so that the distal flaps protrude outwards from the outer ends of the base.

The base of the closing element is fixed to a waistband of a sanitary article. Since the base of the closing element is not elastic, the area of the elastic band on which the base of the closing element is fixed becomes inelastic and reduces the length of the elastic part of the waistband.

### Object and summary of the invention

The object of the present invention is to provide a closing element with an optimized design so as to increase, all other conditions being equal, the length of the elastic part of the waistbands of the sanitary articles.

According to the present invention, this object is achieved by a closing element having the characteristics forming the subject of claim 1.

The claims form an integral part of the disclosure provided here in relation to the invention.

### Brief description of the drawings

The present invention will now be described in detail with reference to the attached drawings, given purely by way of non-limiting example, in which:
- Figure 1 is a schematic perspective view of a particular embodiment of a closing element according to the present invention,
- Figure 2 is a schematic cross-section of the closing element of Figure 1, and
- Figure 3 is a schematic perspective view of a particular embodiment of a closing element according to the present invention.

### Detailed description

With reference to the Figures, numeral 10 indicates a closing element for producing reclosable absorbent sanitary articles.

The closing elements 10 are intended to be fixed at spaced apart positions on a continuous elastic band in a process for producing absorbent sanitary articles according to the technique called Cross Direction, as described in EP-A-1523968 by the same applicant.

The closing element 10 comprises a non-woven sheet 12 folded according to a general omega-shaped configuration.

The folded non-woven sheet 12 comprises a generally flat base 14 subdivided into two sections 16 located on opposite sides of a central plane X perpendicular to the base 14.

The folded non-woven sheet 12 comprises two intermediate flaps 18 parallel to the base 14 and located on opposite sides of the central plane X. The intermediate flaps 18 are connected to respective ends of the base 14 by respective first folds 20. The intermediate flaps 18 are permanently fixed to the base 14, for example, by respective layers of glue 22 or, alternatively, by thermal or ultrasonic welding.

The folded non-woven sheet 12 comprises two distal flaps 24 parallel to the respective intermediate flaps 18 and located on opposite sides of the central plane X. The distal flaps 24 are connected to respective ends of the respective intermediate flaps 18 by respective second folds 26. The distal flaps 24 protrude laterally from the base 14 beyond the respective first folds 20.

The two distal flaps 24 are detachably fixed to the respective intermediate flaps 18 by temporary joining areas 28, which are susceptible to breaking when subjected to a light force so as to allow the mutually facing surfaces of the distal flaps 24 and the intermediate flaps 18 to separate. The temporary bonding areas 28 may be formed by thermal or ultrasonic welding or by technical glue dots.

The fastening element 10 comprises at least two surface fastening elements 30 fixed to surfaces of the distal flaps 24 facing away from the base 14. The surface fastening elements 30 may be micro-hook fastening elements designed to form a Velcro^{®}-type surface closure with corresponding micro-loop fastening elements.

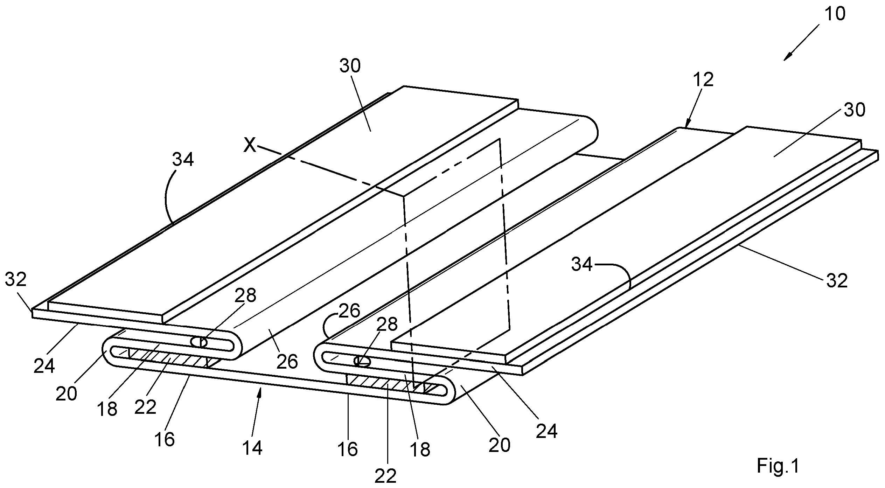

Referring to Figure 1, for each distal flap 24 the surface fastening elements 30 may be continuous along a direction parallel to the central plane X.

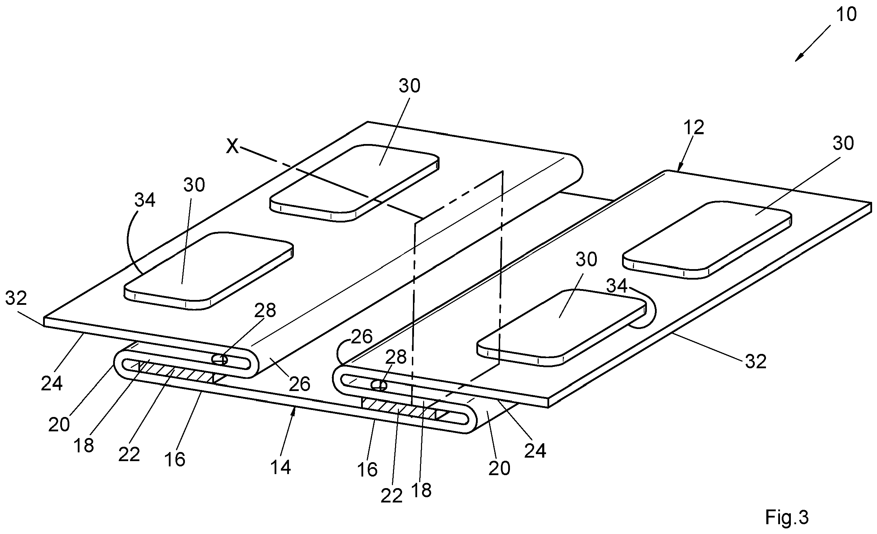

Referring to Figure 3, for each distal flap 24 the surface fastening elements 30 may be discontinuous along a direction parallel to the central plane X.

In accordance with particular embodiments, the surface fastening elements 30 may be made entirely implemented on surfaces portions of the distal flaps 24 opposite to the base 14 by means of, purely by way of example and not limitative, an ultrasonic apparatus such as that described in the patent application EP4108220 by the same Applicant.

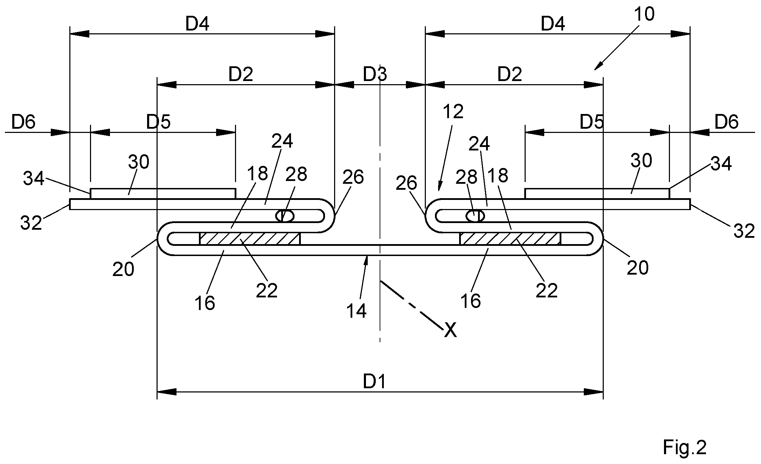

With reference to Figure 2, the base 14 has a dimension D1 in a direction perpendicular to the central plane X equal to the distance between the first folds 20 and between 15 and 90 mm.

Each of the intermediate flaps 18 has a dimension D2 in a direction perpendicular to the central plane X equal to the distance between a first fold 20 and a second fold 26 and between 5 and 30 mm.

The dimension D3 in a direction perpendicular to the central plane X equal to the distance between the second folds 26 is between 5 and 30 mm.

The distance between each second fold 26 and the central plane X, equal to D3/2, is less than the dimension D2 of each intermediate flap 18, or rather: D3/2 < D2.

Each distal flap 24 has a dimension D4 in a direction perpendicular to the central plane X equal to the distance between the second fold 26 and a respective end 32 of the distal flap 24 between 10 and 76 mm.

The dimension D4 of each distal flap 24 may be greater than the sum of the dimension D2 of each intermediate flap 18 and half the dimension D1 of the base 14, i.e. D4 > D2 + D1/2.

Each surface fixing element 30 may have a dimension D5 in a direction perpendicular to the central plane X of between 5 and 25 mm.

Each distal flap 24 has a dimension D6 in a direction perpendicular to the central plane X equal to the distance between the end 32 and the outer edge 34 of the corresponding surface fixing element 30 between 3 and 12 mm.

It is understood that for an expert in the field the dimensions D1, D2, D3, D4, D5 and D6 are easily measurable on a closing element 10 of an absorbent sanitary article in accordance with the present invention by suitable length measuring instruments, such as for example a millimeter tape or a caliper.

In particular, an expert in the field would be able to trace the dimension D3 by measuring on a closing element 10 of an absorbent sanitary article in accordance with the present invention the distance between each second fold 26 and the central plane X, equal to D3/2, and doubling its value.

Furthermore, it may be technically reasonable for an expert in the field to determine for the same dimension D1, D2, D3, D4, D5 and D6 a representative value of multiple measurements taken along a direction parallel to the central plane X, such as the arithmetic mean, and compare this representative value with the respective distance intervals D1, D2, D3, D4, D5 and D6 reported above.

Of course, without prejudice to the principle of the invention, the details of construction and the embodiments can be widely varied with respect to those described and illustrated, without thereby departing from the scope of the invention as defined by the claims that follow.

## Claims

1. A closing element for producing reclosable absorbent sanitary articles, comprising a non-woven sheet (12) folded according to a general omega-shaped configuration and including:
- a base (14) divided into two sections (16) located on opposite sides with respect to a central plane (X) perpendicular to the base (14),
- two intermediate flaps (18) parallel to the base (14) and located on opposite sides of said central plane (X), wherein the intermediate flaps (18) are connected to respective ends of the base (14) by respective first folds (20) and are permanently fixed to the base (14),
- two distal flaps (24) parallel to the respective intermediate flaps (18) and located on opposite sides of said central plane (X), wherein the distal flaps (24) are connected to respective ends of the respective intermediate flaps (18) by respective second folds (26) and are separably fixed to respective intermediate flaps (18), wherein said distal flaps (24) protrude laterally with respect to the base (14) beyond respective first flaps (20),
wherein the fastening element (10) comprises at least two surface fastening elements (30) fixed on surfaces of said distal flaps (24) opposite to the base (14),
**characterized in that** the base (14) has a dimension (D1) in a direction perpendicular to the central plane (X) equal to the distance between the first folds (20) and comprised between 15 and 90 mm and **in that** each of said intermediates flaps (18) has a dimension (D2) in a direction perpendicular to the central plane (X) equal to the distance between a first fold (20) and a second fold (26) and comprised between 5 and 30 mm.

2. The closing element of claim 1, wherein a dimension (D3) in a direction perpendicular to the central plane (X) equal to the distance between the second folds (26) is comprised between 5 and 30 mm.

3. The closing element of claim 2, wherein the distance between each second fold (26) and the central plane (X) is less than the dimension (D2) of each intermediate flap (18).

4. The closing element of any of the preceding claims, wherein each distal flap (24) has a dimension (D4) in a direction perpendicular to the central plane (X) equal to the distance between the second fold (26) and a respective end (32) of the distal flap (24) comprised between 10 and 76 mm.

5. The closing element of claim 4, wherein the dimension (D4) of each distal flap (24) is greater than the sum of the dimension (D2) of each intermediate flap (18) and half the dimension (D1) of the base (14).

6. The closing element of any one of the preceding claims, wherein said at least one surface fastening element (30) has a dimension (D5) in a direction perpendicular to the central plane (X) comprised between 5 and 25 mm.

7. The closing element of claim 6, wherein in each distal flap (24) a dimension (D6) in a direction perpendicular to the central plane (X) equal to the distance between the end (32) and the outer edge (34) of the corresponding surface fastening element (30) is comprised between 3 and 12 mm.

8. The closing element of any of the preceding claims, wherein for each distal flap (24) the surface fastening elements (30) are discontinuous along a direction parallel to the central plane (X).

## Patentansprüche

1. Ein Verschlusselement zur Herstellung wiederverschließbarer absorbierender Hygieneartikel, umfassend eine Vliesbahn (12), die gemäß einer allgemeinen omega-förmigen Konfiguration gefaltet ist und umfasst:
- eine Basis (14), die in zwei Abschnitte (16) unterteilt ist, die sich auf gegenüberliegenden Seiten in Bezug auf eine zentrale Ebene (X) senkrecht zur Basis (14) befinden,
- zwei Zwischenlaschen (18) parallel zur Basis (14) und angeordnet auf gegenüberliegenden Seiten der genannten zentralen Ebene (X), wobei die Zwischenlaschen (18) mit jeweiligen Enden der Basis (14) durch jeweilige erste Falten (20) verbunden und dauerhaft an der Basis (14) befestigt sind,
- zwei distale Laschen (24) parallel zu den jeweiligen Zwischenlaschen (18) und angeordnet auf gegenüberliegenden Seiten der genannten zentralen Ebene (X), wobei die distalen Laschen (24) mit jeweiligen Enden der jeweiligen Zwischenlaschen (18) durch jeweilige zweite Falten (26) verbunden und trennbar an jeweiligen Zwischenlaschen (18) befestigt sind, wobei die genannten distalen Laschen (24) seitlich in Bezug auf die Basis (14) über jeweilige erste Falten (20) hinausragen,
wobei das Befestigungselement (10) mindestens zwei Oberflächenbefestigungselemente (30) umfasst, die auf Oberflächen der genannten distalen Laschen (24) gegenüber der Basis (14) befestigt sind,
**dadurch gekennzeichnet, dass** die Basis (14) eine Abmessung (D1) in einer Richtung senkrecht zur zentralen Ebene (X) gleich dem Abstand zwischen den ersten Falten (20) aufweist und zwischen 15 und 90 mm beträgt und dass jede der genannten Zwischenlaschen (18) eine Abmessung (D2) in einer Richtung senkrecht zur zentralen Ebene (X) gleich dem Abstand zwischen einer ersten Falte (20) und einer zweiten Falte (26) aufweist und zwischen 5 und 30 mm beträgt.

2. Das Verschlusselement nach Anspruch 1, wobei eine Abmessung (D3) in einer Richtung senkrecht zur zentralen Ebene (X) gleich dem Abstand zwischen den zweiten Falten (26) zwischen 5 und 30 mm beträgt.

3. Das Verschlusselement nach Anspruch 2, wobei der Abstand zwischen jeder zweiten Falte (26) und der zentralen Ebene (X) kleiner ist als die Abmessung (D2) jeder Zwischenlasche (18).

4. Das Verschlusselement nach einem der vorhergehenden Ansprüche, wobei jede distale Lasche (24) eine Abmessung (D4) in einer Richtung senkrecht zur zentralen Ebene (X) gleich dem Abstand zwischen der zweiten Falte (26) und einem jeweiligen Ende (32) der distalen Lasche (24) aufweist, die zwischen 10 und 76 mm beträgt.

5. Das Verschlusselement nach Anspruch 4, wobei die Abmessung (D4) jeder distalen Lasche (24) größer ist als die Summe der Abmessung (D2) jeder Zwischenlasche (18) und der Hälfte der Abmessung (D1) der Basis (14).

6. Das Verschlusselement nach einem der vorhergehenden Ansprüche, wobei das genannte mindestens eine Oberflächenbefestigungselement (30) eine Abmessung (D5) in einer Richtung senkrecht zur zentralen Ebene (X) zwischen 5 und 25 mm aufweist.

7. Das Verschlusselement nach Anspruch 6, wobei in jeder distalen Lasche (24) eine Abmessung (D6) in einer Richtung senkrecht zur zentralen Ebene (X) gleich dem Abstand zwischen dem Ende (32) und der Außenkante (34) des entsprechenden Oberflächenbefestigungselements (30) zwischen 3 und 12 mm beträgt.

8. Das Verschlusselement nach einem der vorhergehenden Ansprüche, wobei für jede distale Lasche (24) die Oberflächenbefestigungselemente (30) entlang einer Richtung parallel zur zentralen Ebene (X) diskontinuierlich sind.

## Revendications

1. Élément de fermeture pour produire des articles sanitaires absorbants refermables, comprenant une feuille non tissée (12) pliée selon une configuration générale en forme d'oméga et incluant :
- une base (14) divisée en deux sections (16) situées sur des côtés opposés par rapport à un plan central (X) perpendiculaire à la base (14),
- deux volets intermédiaires (18) parallèles à la base (14) et situés sur des côtés opposés dudit plan central (X), dans lequel les volets intermédiaires (18) sont reliés à des extrémités respectives de la base (14) par des premiers plis respectifs (20) et sont fixés de manière permanente à la base (14),
- deux volets distaux (24) parallèles aux volets intermédiaires respectifs (18) et situés sur des côtés opposés dudit plan central (X), dans lequel les volets distaux (24) sont reliés à des extrémités respectives des volets intermédiaires respectifs (18) par des deuxièmes plis respectifs (26) et sont fixés de manière séparable aux volets intermédiaires respectifs (18), dans lequel lesdits volets distaux (24) font saillie latéralement par rapport à la base (14) au-delà des premiers plis respectifs (20),
dans lequel l'élément de fermeture (10) comprend au moins deux éléments de fixation de surface (30) fixés sur des surfaces desdits volets distaux (24) opposées à la base (14),
**caractérisé en ce que** la base (14) a une dimension (D1) dans une direction perpendiculaire au plan central (X) égale à la distance entre les premiers plis (20) et comprise entre 15 et 90 mm et **en ce que** chacun desdits volets intermédiaires (18) a une dimension (D2) dans une direction perpendiculaire au plan central (X) égale à la distance entre un premier pli (20) et un deuxième pli (26) et comprise entre 5 et 30 mm.

2. Élément de fermeture selon la revendication 1, dans lequel une dimension (D3) dans une direction perpendiculaire au plan central (X) égale à la distance entre les deuxièmes plis (26) est comprise entre 5 et 30 mm.

3. Élément de fermeture selon la revendication 2, dans lequel la distance entre chaque deuxième pli (26) et le plan central (X) est inférieure à la dimension (D2) de chaque volet intermédiaire (18).

4. Élément de fermeture selon l'une quelconque des revendications précédentes, dans lequel chaque volet distal (24) a une dimension (D4) dans une direction perpendiculaire au plan central (X) égale à la distance entre le deuxième pli (26) et une extrémité respective (32) du volet distal (24) comprise entre 10 et 76 mm.

5. Élément de fermeture selon la revendication 4, dans lequel la dimension (D4) de chaque volet distal (24) est supérieure à la somme de la dimension (D2) de chaque volet intermédiaire (18) et de la moitié de la dimension (D1) de la base (14).

6. Élément de fermeture selon l'une quelconque des revendications précédentes, dans lequel ledit au moins un élément de fixation de surface (30) a une dimension (D5) dans une direction perpendiculaire au plan central (X) comprise entre 5 et 25 mm.

7. Élément de fermeture selon la revendication 6, dans lequel dans chaque volet distal (24) une dimension (D6) dans une direction perpendiculaire au plan central (X) égale à la distance entre l'extrémité (32) et le bord extérieur (34) de l'élément de fixation de surface correspondant (30) est comprise entre 3 et 12 mm.

8. Élément de fermeture selon l'une quelconque des revendications précédentes, dans lequel pour chaque volet distal (24) les éléments de fixation de surface (30) sont discontinus le long d'une direction parallèle au plan central (X).
